# EUROPEAN PATENT APPLICATION

(11) **EP 4 509 818 A1**
(43) Date of publication of application: **19.02.2025**
(21) Application number: 23788346.7
(22) Date of filing: 12.04.2023
(51) Int. Cl.: G01N 21/17, B07C 5/342

(54) **DETERMINATION DEVICE, SELECTION DEVICE, AND DETERMINATION METHOD**

(30) Priority: 14.04.2022 JP 2022067081
(71) Applicant: Kabushiki Kaisha Earthtechnica, Tokyo 101-0051 (JP); Toyokin Co., Ltd., Toyota-city, Aichi 471-0836 (JP)
(72) Inventor: TAKANAMI, Hirotoshi, Tokyo 1010051 (JP); KAMINOTA, Shigenori, Tokyo 1010051 (JP); HONGO, Akihiro, Tokyo 1010051 (JP); KURASHIMA, Kazuki, Tokyo 1010051 (JP); NISHIO, Shoichi, Tokyo 1010051 (JP)
(74) Representative: Arnold & Siedsma
(86) International application number: PCT/JP2023/014795
(87) International publication number: WO 2023/199928

(57) **Abstract**

A determination device includes a camera and a controller. The camera photographs a shredded piece and generates a shredded piece image including the shredded piece. The controller determines the content percentage of the additive metal in the shredded piece shown in the shredded piece image based on the shredded piece image generated by the camera. The controller has a determination model constructed by machine learning of shredded piece images and the index indicating the content percentage of the additive metal in the shredded piece shown in the shredded piece image. The controller determines the content percentage of the additive metal in the shredded piece based on the output result obtained by inputting the shredded piece image generated by the camera into the determination model.

## Description

### Technical Field

The present application mainly relates to a determination device for determining content percentage of additive metal in shredded pieces.

### Background Art

Conventionally, there has been known a technique for determining content percentage of additive metals in shredded piece for the purpose of reusing the shredded pieces. A patent literature 1 discloses such a device.

The patent literature 1 describes a method of calculating a surface differential parameter, which is an index relating to the number of times the unevenness of the surface of the shredded pieces changes, by measuring the three-dimensional shape of the surface of the shredded piece. The patent literature 1 describes generating a recognition algorithm by learning surface differential parameters, weight, volume, and the like as parameters. The discrimination algorithm determines whether shredded pieces are shredded pieces of carbon steel or shredded piece of special steel.

### Citation List

### Patent Literature

PTL 1: Japanese Patent No. 6425243

### Summary of Invention

### Technical Problem

In the method described in the patent literature 1, in addition to measuring the three-dimensional shape and calculating the surface differential parameters, it is also necessary to calculate or measure the weight, volume, etc. of the shredded pieces. This complicates the creation of identification algorithms and the process of determining shredded pieces. The patent literature 1 describes that a method of sorting shredded pieces based on differences in appearance using image processing has insufficient sorting accuracy because the shredded pieces are similar in color tone and shape.

This application has been made in view of circumstances described above, and aims primarily to provide a determination device that can determine additive metal content percentage of shredded pieces with high accuracy and has simple processes related to learning and determination. Solution to Problem

The foregoing has described problems to be solved by the present invention. The following will describe solutions to the problems and advantageous effects thereof.

An aspect of the present invention provides a determination device configured as follows. That is, the determination device includes a camera and a controller. The camera photographs a shredded piece and generates a shredded piece image including the shredded piece. The controller determines content percentage of additive metal in the shredded piece shown in the shredded piece image based on the shredded piece image generated by the camera. The controller has a determination model constructed by machine learning of shredded piece images and index indicating the content percentage of the additive metal in the shredded piece shown in the shredded piece image. The controller determines the content percentage of the additive metal in the shredded piece based on output result obtained by inputting the shredded piece image generated by the camera into the determination model.

An aspect of the present invention provides a determination method as follows. That is, a shredded piece of metal is photographed and a shredded piece image including the shredded piece is generated. Content percentage of additive metal in the shredded piece shown in the shredded piece image is determined based on the shredded piece image generated by the camera. The determination is performed by using a determination model constructed by machine learning of the shredded piece images and index indicating the content percentage of the additive metal in the shredded piece shown in the shredded piece image. The content percentage of the additive metal in the shredded piece is determined based on output result obtained by inputting the shredded piece image generated by the camera into the determination model.

### Advantageous Effect of Invention

According to the present application, it is possible to realize a determination device that can determine additive metal content percentage of shredded pieces with high accuracy and has simple processes related to learning and determination.

### Brief Description of Drawings

FIG. 1 is a diagram showing a configuration of a sorting device.
FIG. 2 is a diagram showing a process for constructing a determination model.
FIG. 3 is a diagram showing each layer of the determination model.
FIG. 4 is a graph showing a frequency distribution of a final feature value.
FIG. 5 is a graph showing a frequency distribution of final output value.
FIG. 6 is a flowchart showing processes of determining whether a piece belongs to a low content group or a high content group using the determination model.

### Description of Embodiments

The following will describe, with reference to the drawings, an embodiment of the present invention.

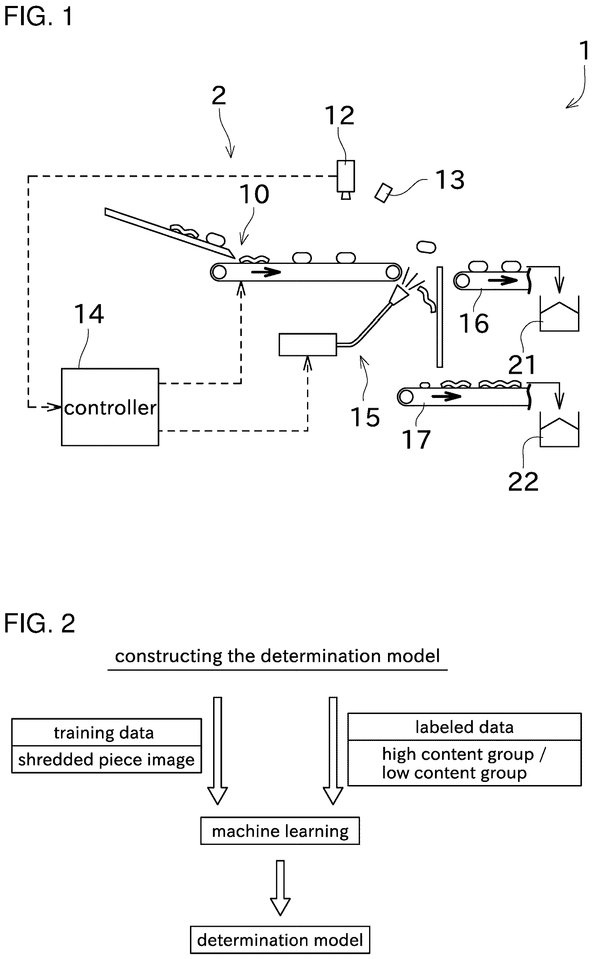

A sorting device 1 shown in FIG. 1 is a device for sorting shredded pieces, which are objects, according to content percentage of additive metals. The shredded pieces can be obtained, for example, by processing metals for recycle metals using a crusher or the like. The crusher is disposed upstream of the sorting device 1, and shredded pieces are supplied to the sorting device 1. The crusher may be located in a factory or the like separate from the sorting device 1. In this case, the shredded pieces produced by the crusher are transported and supplied to the sorting device 1.

The technical content described in this embodiment can also be applied to an apparatus that only determines the content percentage of the additive metal and does not perform sorting. An example of the apparatus that does not perform sorting is a apparatus that measures or inspects shredded pieces.

As shown in FIG. 1, the sorting device 1 includes a conveying device 10, a camera 12, an illuminator 13, a controller 14, and a sorting unit 15. The sorting device 1 also includes a determination device 2 that determines the shredded pieces placed on the conveying device 10. The determination device 2 is composed of the camera 12, the illuminator 13, and the controller 14.

On the upstream side of the conveying device 10, there is a supply source that sequentially supplies a plurality of shredded pieces. The conveying device 10 conveys the shredded pieces supplied from the supply source downstream. The conveying device 10 in this embodiment is a belt conveyor, but another conveying device can be used as the conveying device 10 as long as it is capable of conveying shredded pieces. Another conveying device is, for example, a roller conveyor.

The camera 12 is disposed above the conveying device 10. The camera 12 photographs the shredded piece being conveyed by the conveying device and generates an image including the shredded piece. Hereinafter, an image containing shredded piece will be referred to as a shredded piece image. The camera of this embodiment has a rectangular photographing area. Alternatively, a line camera with a linear photographing area may be used.

The illuminator 13 irradiates the shredded piece photographed by the camera 12 with illumination light. By irradiating the shredded piece with illumination light, the images of the shredded piece captured by the camera 12 can be made clearer. The illuminator 13 is disposed above the conveying device 10 and downstream of the camera 12 in the conveying direction. The disposition of the illuminator 13 is merely an example, and the illuminator 13 may be arranged, for example, at a position upstream of the camera 12 in the conveying direction.

The sorting device 1 sorts the shredded pieces into two levels according to the content percentage of additive metals. Hereinafter, the group with a higher content percentage of additive metals will be referred to as a high content group, and the group with a lower content percentage of additive metals will be referred to as a low content group. The grouping is not limited to two levels, but may be three or more levels. For example, if the shredded pieces are steel and contain large amounts of additive metals such as manganese or chromium, they may not be suitable for use as cast iron for recycling purposes. For example, steel with high manganese or chromium content may be less likely to precipitate graphite during solidification in casting, or may produce a brittle structure called chill. Therefore, by sorting the shredded pieces according to the content percentage of additive metals, it is possible to separate steel suitable for recycling purposes from steel unsuitable for a recycling purpose. The composition of the shredded pieces is not limited to steel, but may be another alloy. Another alloys is, for example, iron alloy other than steel or aluminum alloy. The additive metal is not limited to manganese or chromium, but may be, for example, nickel.

The controller 14 includes an arithmetic unit such as an FPGA, an ASIC, or a CPU, and a storage device such as an SSD or a flash memory. The arithmetic unit executes various processes related to the sorting device 1 by reading and executing programs stored in the storage device. The controller 14 determines, for example, based on the shredded piece image, whether the shredded piece belongs to the high content group or the low content group. The details of this determination will be described later. The controller 14 further controls the conveying device 10 and the sorting unit 15.

The sorting unit 15 is configured to be able to switch between sending the shredded pieces transported by the conveying device 10 to a first collector 21 via a first conveying device 16 or to a second collector 22 via a second conveying device 17. The first collector 21 is a container that mainly collects shredded pieces of the low content group. The second collector 22 is a container that mainly collects shredded pieces of the high content group. The sorting unit 15 can send the shredded piece to the first conveying device 16 by spraying compressed air toward the shredded piece. When the sorting unit 15 does not spray compressed air, the shredded piece fall naturally and is sent to the second conveying device 17. The position and timing at which the sorting unit sprays compressed air is controlled by the controller 14.

The sorting unit 15 may sort the shredded piece of the high content group by spraying compressed air. The sorting unit 15 is not limited to a machine that sprays compressed air. For example, the sorting unit 15 may be an arm robot. The arm robot picks up the shredded piece and places it in different locations depending on whether the shredded piece belongs to the high content group or the low content group.

Next, processes of classifying the shredded pieces shown in the shredded piece image into a high content group and a low content group based on the shredded piece image will be described.

The controller 14 has a determination model that is constructed in advance by performing machine learning. As shown in FIG. 2, the determination model is constructed by machine learning of the training data and the labeled data. In this embodiment, the training data consists shredded piece images, and the labeled data is whether the shredded piece belongs to the high content group or the low content group. This machine learning corresponds to supervised learning because it involves the presentation of the labeled data.

It is preferable that the shredded piece images used as training data are shredded piece images generated using the sorting device 1. This allows the imaging conditions to be consistent when constructing the determination model and when the sorting device 1 is in operation, thereby improving determination accuracy. However, training data may be generated in an environment or with a camera different from that of the sorting device 1. A certain shredded piece image may be used as training data, and an image obtained by rotating this shredded piece image may be used as separate training data. This makes it possible to prepare a large amount of training data with simple processing.

The labeled data may be an index showing the content percentage of the additive metal in the shredded pieces, and is not limited to a high content group or a low content group. For example, when the shredded pieces are sorted into three or more groups according to the content percentage of the additive metal, it is preferable to classify the labeled data into three or more groups. An estimated value or a measured value of the content percentage of the additive metal may be used as the labeled data.

Since the machine learning in this embodiment is deep learning, it is not necessary to extract and input features from the shredded piece image, and it is sufficient to simply input the shredded piece image itself. In order to obtain a valid conclusion using the determination model, it is necessary that there is a correlation between the shredded piece image and the content percentage of the additive metal. Therefore, in the following, it will be explained that the content percentage of the additive metal can be determined based on the shredded piece image.

The additive metal is added in order to improve the strength of an iron alloy or the like. Therefore, shredded pieces having a high content percentage of the additive metal are relatively strong and are less likely to deform when processed by a crusher. Conversely, shredded pieces having a low content percentage of the additive metal have relatively low strength and are easily deformed when processed by a crusher. Specifically, the lower the content percentage of the additive metal in a steel plate, the more easily it is bent into a rounded shape when fed into a crusher, and as a result, wrinkles are more likely to appear on the surface of the shredded pieces.

Therefore, there is a correlation between the wrinkle ratio, which is the ratio of the surface of the shredded piece that is wrinkled, and the content percentage of the additive metal. Specifically, the higher the wrinkle ratio, the lower the content percentage of the additive metal. Furthermore, since wrinkles appear in the shredded piece image, it is possible to determine the content percentage of the additive metal based on the shredded piece images.

However, since the size and surface shape of the shredded pieces vary, and the light hitting them is not uniform, it is difficult to accurately determine the wrinkle ratio using a predetermined calculation. In this regard, by using the above-mentioned determination model, it is possible to flexibly take into account differences in the tendencies of the degree of wrinkles of shredded pieces in the high content group and the low content group, thereby achieving high determination accuracy.

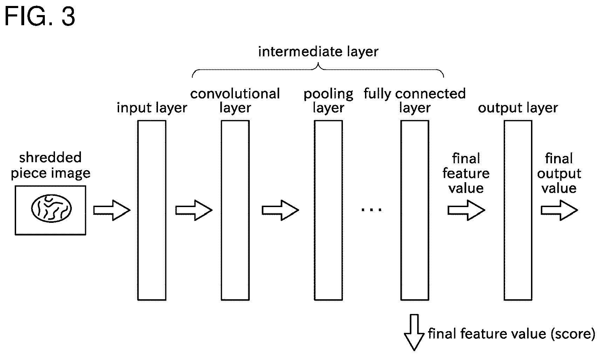

Next, the determination model will be described in more detail. As shown in FIG. 3, the determination model includes an input layer, intermediate layers, and an output layer. Moreover, the determination model of this embodiment is constructed using a convolutional neural network. Therefore, the intermediate layers include a convolutional layer, a pooling layer, and a fully connected layer. The determination model is not necessarily constructed using the convolutional neural network, and it may be constructed using other neural networks.

The shredded piece images are input to the input layer. In the convolutional layers, images with specific features extracted are generated by using filters to extract particular features from an image. The type of features extracted varies depending on the filter. The pooling layer compresses information by downsampling the image that has extracted particular features. The determination model includes multiple sets of convolutional layers and pooling layers, and various features are extracted through these layers.

The fully connected layer receives the output values from each pooling layer. The fully connected layer outputs the final feature value based on the output from each pooling layer. The final feature value are input to the output layer. The final feature value includes a first element that is a numerical representation of the likelihood of belonging to the low content group, and a second element that is a numerical representation of the likelihood of belonging to the high content group.

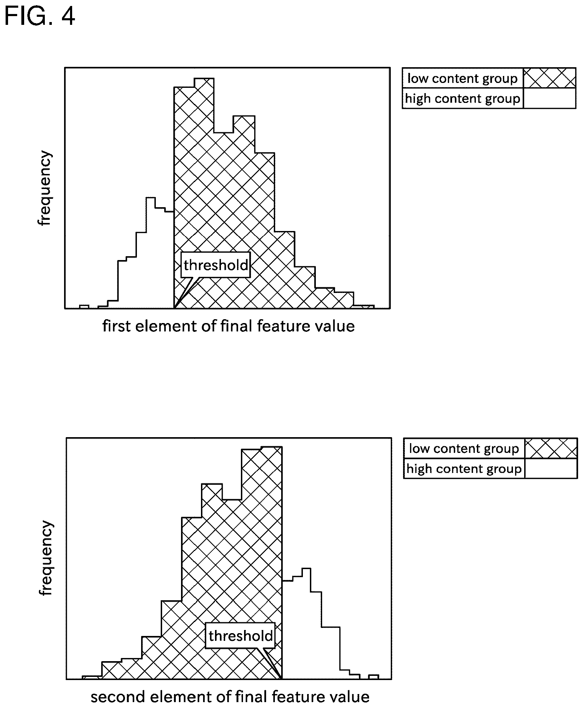

FIG. 4 shows the frequency distribution of the first element and the second element of the final feature value. The upper graph is for the first element of the final feature value, with the horizontal axis representing the value of the first element and the vertical axis representing the frequency. The lower graph is for the second element of the final feature value, with the horizontal axis representing the value of the second element and the vertical axis representing the frequency. The threshold will be described later.

In the output layer, processing is performed using an activation function. The activation function is, for example, the softmax function. By performing processing using the activation function, the final feature value is converted into the final output value. The final output value includes a first element indicating the probability of belonging to the low content group and a second element indicating the probability of belonging to the high content group.

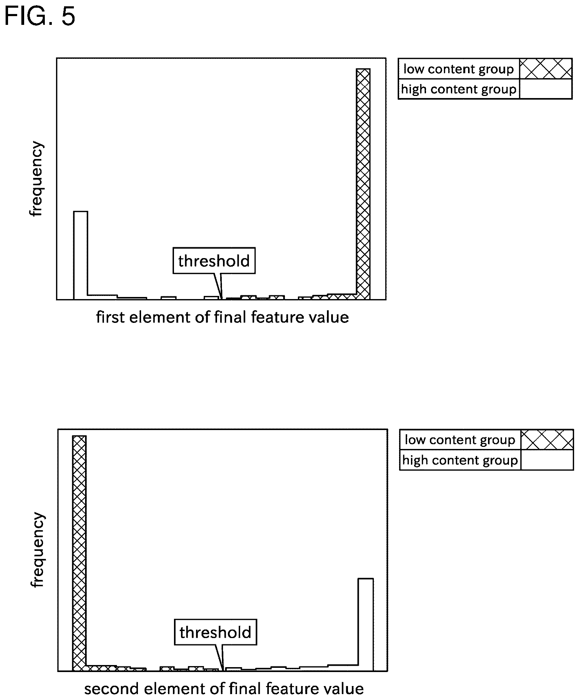

FIG. 5 shows the frequency distribution of the first and second elements of the final output value. The upper graph is for the first element of the final output value, with the horizontal axis representing the value of the first element and the vertical axis representing the frequency. The lower graph relates to the second element of the final output value, with the horizontal axis representing the value of the second element and the vertical axis representing the frequency.

Here, there is a need to change the boundary between the high content group and the low content groups of the shredded pieces after the sorting device 1 has been in operation. For example, a change in the boundary may be desired if the type of shredded pieces changes, if the delivery destination of recycled products changes, or if there is a desire to increase the amount of crushed products classified as low content group.

Since the determination model of the this embodiment calculates the final feature value or the final output value, the boundary between the high content group and the low content group can be changed by setting a threshold value for these values. However, as shown in FIG. 5, the final output values are distributed in a concentrated manner in a certain range, so that even if the threshold value for the final output value is changed, the determination result does not change much. In contrast, as shown in FIG. 4, the final feature values are distributed over a wide range. Therefore, by changing the threshold value for the final feature value, the boundary between the high content group and the low content group is changed, and a new determination result different from that before the threshold value was changed can be obtained. The threshold value can be changed, for example, by an operator operating the controller 14 or a device capable of communicating with the controller 14 to input the threshold value.

Therefore, in this embodiment, the final feature value output by the fully connected layer is extracted as a score, and based on the score, it is determined whether the feature belongs to the high content group or the low content group. Either the first element or the second element may be used as the score, but in this embodiment, the first element is used as the score.

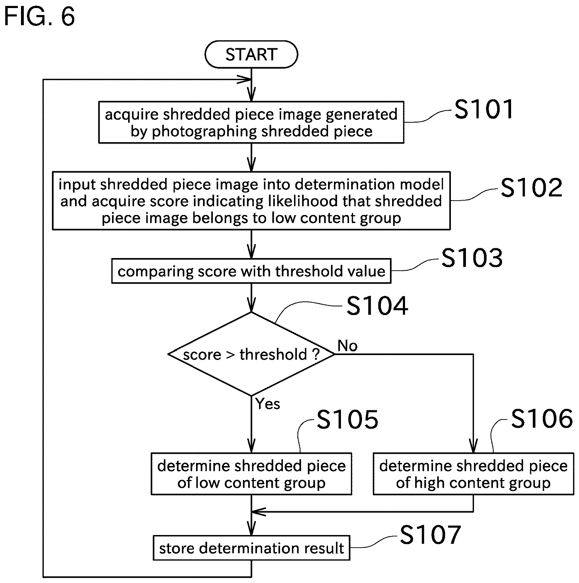

Next, the process performed by the controller 14 will be described with reference to FIG. 6.

The controller 14 acquires, from the camera 12, shredded piece image that the camera 12 generates by photographing the shredded piece (S101). Next, the controller 14 inputs the shredded piece image into the determination model and obtains a score indicating the likelihood that the shredded piece image belongs to the low content group (S102). As described above, the score is the first element of the final feature value, and the higher the probability of belonging to the low content group, the higher the score value.

Next, the controller 14 compares the score with the threshold value (S103). When the controller 14 determines that the score is greater than the threshold value (S104), it determines that the shredded piece shown in the shredded piece image belongs to the low content group (S105). When the controller 14 determines that the score is equal to or less than the threshold value (S104), it determines that the shredded piece shown in the shredded piece image belongs to the high content group (S106).

Next, the controller 14 stores the determination result (S107). Thereafter, the controller 14 sprays the compressed air into the sorting unit 15 at the timing when the shredded piece determined to be in the low content group pass through the sorting unit 15. In this way, the shredded pieces can be sorted according to the content percentage of the additive metal.

As described above, the determination device 2 of this embodiment has the following feature 1. That is, the determination device 2 includes the camera 12 and the controller, and performs the following determination method. The camera 12 photographs the shredded piece and generates the shredded piece image including the shredded piece. The controller 14 determines the content percentage of the additive metal in the shredded piece shown in the shredded piece image based on the shredded piece image generated by the camera 12. The controller 14 has the determination model constructed by machine learning of shredded piece images and the index indicating the content percentage of the additive metal in the shredded piece shown in the shredded piece image. The controller 14 determines the content percentage of the additive metal in the shredded piece based on the output result obtained by inputting the shredded piece image generated by the camera 12 into the determination model.

By using the determination model constructed through machine learning, it is possible to obtain output results that take into account the correlation and trends between the content percentage of the additive metal of the shredded piece of the shredded piece image. Therefore, higher determination accuracy can be achieved compared to a case where a determination model is not used. Additionally, by using the shredded piece image, the processes involved in machine learning and determination can be simplified.

The determination device 2 of this embodiment has the following feature 2. That is, a number of levels are set according to the content percentage of the additive metal in the shredded piece. The controller 14 determines which level the shredded piece shown in the shredded piece image belongs to.

This allows the shredded piece to be classified according to the content percentage of the additive metal, so that different treatments can be performed on the shredded piece depending on the content percentage of the additive metal, for example.

The determination device 2 of this embodiment has the following feature 3. That is, the output result output by the determination model is a score indicating the degree of probability that the shredded piece shown in the shredded piece image belongs to the level.

The score can be used to distinguish whether the shredded piece has a high or low probability of belonging to a certain level.

The determination device 2 of this embodiment has the following feature 4. That is, the threshold is set for each level. The controller 14 determines that the score belongs to the level when the score exceeds the threshold value.

By using the threshold, it is easy to determine whether a shredded piece belongs to the certain level or not.

The determination device 2 of this embodiment has the following feature 5. That is, the controller 14 changes the threshold value based on information input by the operator.

By changing the threshold value, it is possible to adjust the determination criteria for each level according to the requirements of an installation site of the determination device 2, for example.

The determination device 2 of this embodiment has the following feature 6. That is, the determination model is constructed of the neural network including at least the input layer, the intermediate layer, and the output layer. In the output layer, processing is performed using the activation function. The input value to the activation function is used as the score.

The values input to the activation function have a wider range than the output values of the activation function, so that the shredded piece can be appropriately classified according to the threshold value.

The determination device 2 of this embodiment has the following feature 7. That is, the determination model is constructed using the convolutional neural network and includes at least the convolutional layer, the pooling layer, and the fully connected layer. The output value of the fully connected layer is used as the score.

Since the output values of the fully connected layer take a wider range of values than the output values of the activation function, it is possible to appropriately classify the shredded piece according to the threshold value.

The above-mentioned features 1 to 7 can be appropriately combined as long as no contradiction occurs. For example, the feature 3 can be combined with at least one of the features 1 and feature 2. The feature 4 can be combined with at least one of features 1 to 3. The feature 5 can be combined with at least one of features 1 to 4. The feature 6 can be combined with at least one of features 1 to 5. The feature 7 can be combined with at least one of features 1 to 6.

While a preferred embodiment of the present invention has been described above, the configurations described above may be modified, for example, as follows.

The determination model shown in the above embodiment includes the output layer that performs processing using the activation function, but the output layer may be omitted.

The flowchart illustrated in the embodiment described above is just an example, and it may be acceptable that a part of the process is omitted, contents of a part of the process are changed, or a new process is added. For example, processing may be performed using the probability of belonging to the high content group as the score, rather than the probability of belonging to the low content group. Also, the process of determining whether the substance is in the low content group or the high content group may be omitted, and the score may be stored.

The functionality of the elements disclosed herein may be implemented using circuitry or processing circuitry which includes general purpose processors, special purpose processors, integrated circuits, ASICs (Application Specific Integrated Circuits), conventional circuitry and/or combinations thereof which are configured or programmed to perform the disclosed functionality. Processors are considered as processing circuitry or circuitry as they include transistors and other circuitry therein. In the disclosure, the circuitry, units, or means are hardware that carry out the recited functionality, or are hardware programmed to perform the recited functionality. The hardware may be any hardware disclosed herein or otherwise may be other known hardware which is programmed or configured to carry out the recited functionality. When the hardware is a processor which may be considered as a type of circuitry, the circuitry, means, or units are a combination of hardware and software, the software being used to configure the hardware and/or processor.

## Claims

1. A determination device comprising:
a camera that photographs a shredded piece of metal and generates a shredded piece image including the shredded piece; and
A controller that determines content percentage of additive metal in the shredded piece shown in the shredded piece image based on the shredded piece image generated by the camera,
wherein, the controller has a determination model constructed by machine learning of shredded piece images and index indicating the content percentage of the additive metal in the shredded piece shown in the shredded piece image, and
the controller determines the content percentage of the additive metal in the shredded piece based on output result obtained by inputting the shredded piece image generated by the camera into the determination model.

2. The determination device according to claim 1, wherein
a plurality of levels are set according to the content percentage of the additive metal in the shredded piece, and
the controller determines which level the shredded piece shown in the shredded piece image belongs to.

3. The determination device according to claim 2, wherein
the output result output by the determination model is a score indicating a degree of likehood that the shredded piece shown in the shredded piece image belongs to the level.

4. The determination device according to claim 3, wherein
the threshold is set for each level, and
the controller determines that the score belongs to the level when the score exceeds the threshold value.

5. The determination device according to claim 4, wherein
the controller changes the threshold value based on information input by the operator.

6. The determination device according to claim 4 or 5, wherein
the determination model is constructed of a neural network including at least an input layer, an intermediate layer, and an output layer,
in the output layer, processing is performed using an activation function, and
the input value to the activation function is used as the score.

7. The determination device according to claim 4 or 5, wherein
the determination model is constructed of a convolutional neural network and includes at least a convolutional layer, a pooling layer, and a fully connected layer, and
the output value of the fully connected layer is used as the score.

8. The determination device according to claim 1, further comprising
a sorting unit that sorts the shredded piece based on the result of the determination device.

9. A determination method comprising:
photographing a shredded piece of metal and generating a shredded piece image including the shredded piece; and
performing a determination of content percentage of an additive metal in the shredded piece shown in the shredded piece image based on the shredded piece image generated by the camera, wherein
wherein, the determination is performed by using a determination model constructed by machine learning of the shredded piece images and index indicating the content percentage of the additive metal in the shredded piece shown in the shredded piece image, and
the content percentage of the additive metal in the shredded piece is determined based on output result obtained by inputting the shredded piece image generated by the camera into the determination model.
